# EUROPEAN PATENT APPLICATION

(11) **EP 4 201 303 A1**
(43) Date of publication of application: **28.06.2023**
(21) Application number: 21315286.1
(22) Date of filing: 21.12.2021
(51) Int. Cl.: A61B 1/31, A61B 1/00

(54) **INSERTION INSTRUMENT FOR INSERTION INTO A CAVITY**

(71) Applicant: Caranx Medical SAS, 75013 Paris (FR)
(72) Inventor: PRZYBYLSKI, Flavie, 62980 Noyelles-les-vermelles (FR); BERTHET-RAYNE, Pierre, 06800 Cagnes-sur-Mer (FR); SEJOR, Eric, 06000 Nice (FR); SMITS, Jonas Victor Harmen, 3360 Bierbeek (BE)
(74) Representative: Hepp Wenger Ryffel AG

(57) **Abstract**

The invention refers to an insertion instrument (1a-1e), in particular a surgical insertion instrument, for insertion into a cavity, preferably a human cavity. The instrument (1a-1e) comprises:
- a flexible tubular sheath (2) having a proximal annular end (3), a distal annular end (4) and a middle section (5) extendable between the proximal annular end (3) and the distal annular end (4),
- a holding member (6) holding the proximal annular end (3),
- a pressurizing unit for pressurizing the distal annular end (4) of the flexible tubular sheath (2) for everting the sheath (2) at the distal annular end (4).

The pressurizing unit comprises:
- a fluid supply member and
- a pressure transmittal head (7) being movably arranged in the tubular sheath (2) and in proximity to the distal annular end (4), preferably at the distal annular end (4), wherein the pressure transmittal head (7) is in fluid connection with the fluid supply member by means of a fluid transmission path (8).

The distal annular end (4) and the pressure transmittal head (7) form at least one dynamic pressure chamber (9). The flexible tubular sheath (2) is deployable and invertible at the distal annular end (4) upon pressurizing said at least one dynamic pressure chamber (9) by means of the pressure transmittal head (7), thereby extending the middle section (5) of the flexible tubular sheath (2).

## Description

The invention relates to an insertion instrument, in particular a surgical insertion instrument, and a set for insertion into a cavity comprising said insertion instrument according to the preamble of the independent claims.

Pneumatically everting soft robots imitate plant-like growth by extending new material from their tip to navigate their environment. Movement through growth allows these robots to navigate easily through cluttered environments, in particular long and cluttered cavities. Such cavities can be found e.g. in buildings, such as pipelines or cable trays; or in human or animal bodies, in particular the intestine.

On example of such soft robot is described in US 2019/217908 A1, in which a robot for navigating an environment through growth is provided. The growing robot has a thin-walled, hollow, pressurized, compliant body that elongates the body by everting from its tip new wall material that is stored inside the body and controls the shape of the body by actively controlling the relative lengths of the wall material along opposing sides of the body. The tip of the robot is equipped with a camera, which allows exploring of the surrounding environment.

The drawback of such growing robots is their tendency to straighten the surrounding environment. This is in particular a problem if the growing robot is used to pass through the intestine. The straightening of the anatomy bears the risk of injuring the patient.

The straightening is due to the fact that the robot is pressurized all along its body and therefore automatically wants to resume a linear shape since its environment is not fixed and rigid.

The deformation of the intestine can lead to stretching of the blood vessels, which, in turn, can lead to tearing of the blood vessels or the lumen, which may cause severe complication.

There is a need for growing robots that allows examination of soft cavities, such as the intestine, without the risk of straightening the cavity.

It is at least one objective to overcome the drawbacks of the prior art. In particular, it is one objective of the present invention to provide an insertion instrument for insertion into a cavity in which the growing body remains essentially flexible during grows, reducing the risk of straightening of soft cavities.

The at least one objective is solved by the subject-matter of the independent claims. Preferred embodiments are described with regard to the dependent claims.

A first aspect of the invention relates to an insertion instrument, in particular a surgical insertion instrument, for insertion into a cavity, preferably a human cavity. The instrument comprises:
- a flexible tubular sheath having a proximal annular end, a distal annular end and a middle section extendable between the proximal annular end and the distal annular end;
- a holding member holding the proximal annular end;
- a pressurizing unit for pressurizing the distal annular end of the flexible tubular sheath for everting the sheath at the distal annular end.

The pressurizing unit comprises
- a fluid supply member and
- a pressure transmittal head being movably arranged in the tubular sheath and in proximity to the distal annular end, preferably at the distal annular end.

The pressure transmittal head is in fluid connection with the fluid supply member by means of a fluid transmission path. The distal annular end and the pressure transmittal head form at least one dynamic pressure chamber. The flexible tubular sheath is deployable and invertible at the distal annular end upon pressurizing said at least one dynamic pressure chamber by means of the pressure transmittal head, thereby extending the middle section of the flexible tubular sheath.

The insertion instrument is not limited to medical application but may also be used in industrial application, in buildings, in search and rescue, in exploration and the like.

The fluid supply member can refer to an external supply member, e.g. provided .by the operating environment, or can be an integrated part of the device. The integrated fluid supply member can be a distal air pump or a canister/reservoir. A fluid can be a gas or liquid, air is particularly preferred. Air may be used for endoscopy while fluid may be preferred for endovascular examinations.

The integrated air supply member can be directly associated with the pressure transmittal head to bring the air only to the at least one dynamic pressure chamber.

The term "dynamic pressure chamber" refers to a fluid-tight chamber, allowing movement of the head with regard to the sheath with deployment of the sheath and upon pressurizing said chamber.

The flexible tubular sheath may be inflatable.

The insertion instrument allows a functional separation of operating pressure from the tubular body. Only the distal end is pressurized while the rest of the robot can remain unpressurized. This provides a more flexible tubular sheath preventing straightening of soft cavities. This is in particular advantageously for use in medical application. Gastro-intestinal or other anatomical explorations are possible with a strongly reduced risk of any damage, ensuring safety of the patient.

The fluid transmission path may comprise at least one, preferably two, unrollable hose(s) connected to the pressure transmittal head for delivering fluid from the fluid supply member, preferably the external fluid supply member, to the pressure transmittal head. The hoses can be made of a soft, flexible material, similar to the tubular sheath of the insertion instrument or even the same material. The hoses can be connected to an air compressor with a specific pressure in order to deploy the sheath from the distal annular end.

The unrollable hose(s) can be arranged on a rotatable base station, or alternatively, can be stored linearly.

In case of an integrated fluid supply member the fluid transmission line may comprise at least one, preferably two, pipelines, e.g. arranged within in the pressure transmittal head, providing a fluid connection between the integrated supply member and the at least one dynamic pressure chamber.

Advantageously, the pressure transmittal head comprises a hollow cylinder with a first inner surface and a first outer surface. The flexible tubular sheath can be slidable arranged over the first inner surface and the first outer surface to slide towards the proximal end.

Preferably, the flexible tubular sheath is sealingly arranged to the first inner surface and to the first outer surface to form - the at least one dynamic pressure chamber.

The pressure transmittal head can comprise a second cylinder with a second outer surface. The flexible tubular sheath can be slidable arranged over the second outer surface to slide towards the proximal end.

The flexible tubular sheath may be sealingly arranged over the second outer surface to form the at least one dynamic pressure chamber.

The pressure transmittal head may comprises apertures for the hoses or pipelines in the hollow cylinder and/or the second cylinder.

The integrated fluid supply member can be associated with the hollow cylinder and/or the second cylinder.

The sealing arrangement between the tubular sheath and outer surface (s) on the pressure transmittal head is not limited to a cylindric configuration but may also rely on conventional sealing configurations used in dynamic sealing of linear motion such as hydraulic actuators, plunger or piston pumps, servo valves, etc., including: O-rings, X-rings, crosshead seals or spring-tensioned lip seals such as a double lip seals with garter springs.

Alternatively, a custom sealing configuration may be devised using the same principles of the above, e.g. sealing lips radially outwards towards the tubular sheath.

Materials could include silicone rubber, PFTE, or other biocompatible and sterilizable elastomers for the sealing materials, and surgical-grade stainless steel for any spring materials, e.g. AISI 304.

Advantageously, the distal annular end and the pressure transmittal head form at least two dynamic pressure chambers which are pressurizable with different pressures. The chambers preferably extend in a longitudinal direction between the distal end and the middle section.

Different pressurization allows bending of the tip in preferred directions.

Pressure differences can be obtained with two separate pressure supplies or with a single pressure supply. A single pressure supply can be supplied to the head and separated into two pressure paths e.g. by adding a component such as a pressure relief or a pressure control valve in the head between the pressure transmission path and the individual volumes used for everting the sheath. In case of two separate pressure supplies, two separate pressure lines can be provided with two compressors or one compressor and two pressure regulators.

In a further preferred embodiment, a means for transmitting a control signal from the holding member to the pressure transmittal head is provided to operate the pressure control valve or regulators, either wirelessly or via an electrical signal cable.

The number of chambers may be adjustable. For example, a sealing arrangement with a triangle shaped ring may be provided to obtain three pressure chambers, preferably pressurizable with two or three different pressures. Thus, more flexibility on the movement of the device, and in particular the head, is provided.

Further, the instrument may comprise a fluid extraction unit, allowing the fluid, preferably air, being removed from the pressurized distal annular end.

The fluid extraction unit may comprise at least one fluid extraction chamber and at least one fluid extraction member in fluid connection with the at least one fluid extraction chamber.

Such a configuration allows an independent depressurization of the sheath and the instrument can be retracted from the cavity.

The insertion instrument can be retracted from the cavity by pulling on the device itself or by pulling on the hoses, or by pulling on an instrument channel or by having a retraction unit inside the head, the retraction unit may be comprise the same components as the fluid extraction unit.

Most preferably, the at least one dynamic pressure chamber is in fluid connection with the at least one fluid extraction chamber. Air may be filled in the pressure chamber and can be extracted through the extraction chamber.

The flexible tubular sheath can be made of a plastic film, preferably LDPE or LLDPE; a coated fabric or a flexible resin. The sheath may be a thin-walled extruded tube or co-joint sheaths via laser welding. Fabric material may also be woven and optionally roll coated. As mentioned further above, the hoses may be made from the same material. If the sheath or hose(s) are made from a resin material, they may be even be obtained with flexible 3D resin printers.

Advantageously, the non-deployed tubular sheath is wounded up inside a separate reservoir, preferably within the pressure transmittal head. Thus, the non-deployed sheath can be easily stored and simplifying the design of the insertion instrument.

Preferably, the flexible tubular sheath has a diameter in the range of 0.1 to 0.8 cm for endovascular use and 0.1 to 2.5 cm for endoscopy.

Preferably, the flexible tubular sheath has a length of up to 1.2 meters for endovascular use and a length up to 9 meters for endoscopy.

Advantageously, a lumen is provided at least in the centre of the distal annular end for accommodating an interior device, preferably a surgical, therapeutic or diagnostic device, sensors, robots, in particular autonomous robots or magnetically guided robots, cameras and drug delivery systems.

Preferably, the lumen is provided as an interior device channel from the proximal end to the distal end through which the interior device can be inserted.

A second aspect of the invention refers to a set for insertion into a cavity, preferably a human cavity. The set comprises an insertion instrument as previously described and at least one interior device, preferably provided at a distal annular end of the insertion instrument. The interior device is in particular selected from the group consisting of surgical, therapeutic or diagnostic instruments, sensors, robots, in particular autonomous robots or magnetically guided robots, cameras and drug delivery systems.

The interior device may be provided though an interior device channel from the proximal end to the distal end as described above. Alternatively, the interior device may be provided on the outside wall of the insertion instrument.

The invention will be described in more detail with the aid of figures. Identical reference signs correspond to identical structural components. The figures are merely illustrative examples and are not to be understood as limiting examples. It shows:
- Figure 1:: A perspective view inside a first embodiment of an insertion instrument according to the invention.
- Figure 2:: Detailed view of the pressure transmittal head of the fist embodiment.
- Figure 3:: A detailed view of a pressure transmittal head according to a second embodiment.
- Figure 4:: Individual steps of the deployment method of the tubular sheath in the insertion instrument according to the first embodiment.
- Figure 5:: Individual steps of the deployment method of the tubular sheath in the insertion instrument according to the second embodiment.
- Figure 6:: sectional view of a third embodiment of an insertion instrument according to the invention.
- Figure 7:: a perspective view of a fourth embodiment of an insertion instrument according to the invention.
- Figure 8A:: a perspective view of a fifth embodiment of an insertion instrument according to the invention.
- Figure 8B:: a front view of the insertion instrument of figure 8A.
- Figure 8C:: a sectional view of the insertion instrument of figure 8A along axis A-A in figure 8B.
- Figure 8D:: a sectional view of the insertion instrument of figure 8A along axis B-B in figure 8B.

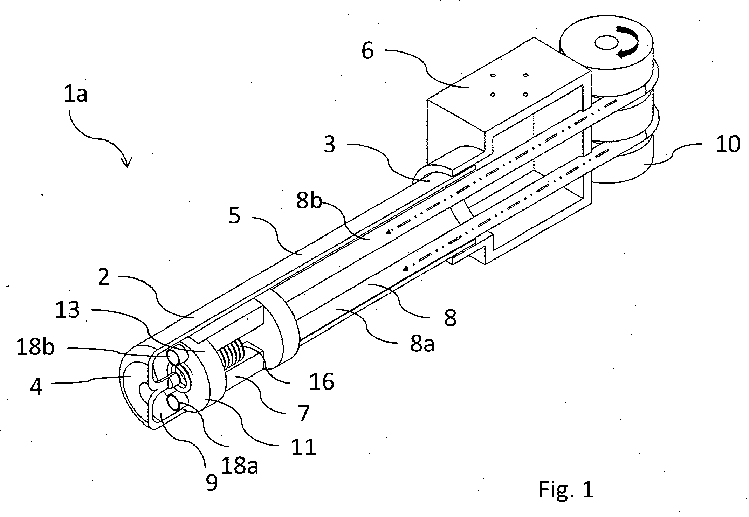

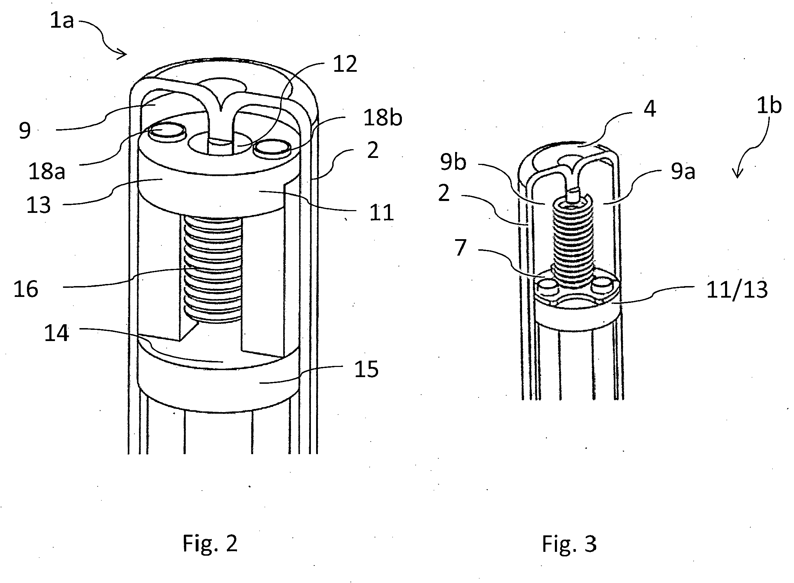

Figure 1 shows a perspective view inside a first embodiment of an insertion instrument 1a according to the invention. The insertion instrument 1a comprises a flexible tubular sheath 2 having a proximal annual end 3, a distal annular end 4 and a middle section 5 extendable between the proximal annular end 3 and the distal annular end 4. The flexible tubular sheath 2 is supported by a holding member 6. A pressure transmittal head 7 is located near the distal annular end 4. Two hoses 8a and 8b provide a fluid transmission path 8 through the flexible tubular sheath 2 to the pressure transmittal head 7. The hoses 8a and 8b are unrollable from a base station 10. The hoses are unrolled in clockwise direction, indicated by the arrow. The pressure transmittal head 7 comprises a first hollow cylinder 11 with a first inner surface (Figure 2) and a first outer surface 13. The first cylinder 11 comprises apertures 18a and 18b which are connected to the hoses 8a and 8b. Between the first cylinder 11 and the distal annular end a dynamic pressure chamber 9 is formed. The dynamic pressure chamber 9 is formed by a sealing interface between the flexible tubular sheath 2 and the first outer surface 13. The pressure transmittal head 7 comprises a second cylinder 14 with a second outer surface 15. A separate reservoir 16 is formed between the first cylinder 11 and the second cylinder 14 in the pressure transmittal head 7 accommodating the deployable flexible tubular sheath 2. The non-deployed tubular sheath 2 is wounded up inside the reservoir.

Figure 2 is a detailed view of the pressure transmittal head 7 of the insertion instrument 1a. The first cylinder 11 is a hollow cylinder with a hole in the centre, providing access to a first internal surface 12 through which the flexible tubular sheath 2 is deployed.

Figure 3 shows a pressure transmittal head 7 according to a second embodiment of an insertion instrument 1b. In this embodiment two dynamic pressure chambers 9a and 9b are formed between the hollow cylinder 11 and the distal annular end 4 which can be pressurized individually.

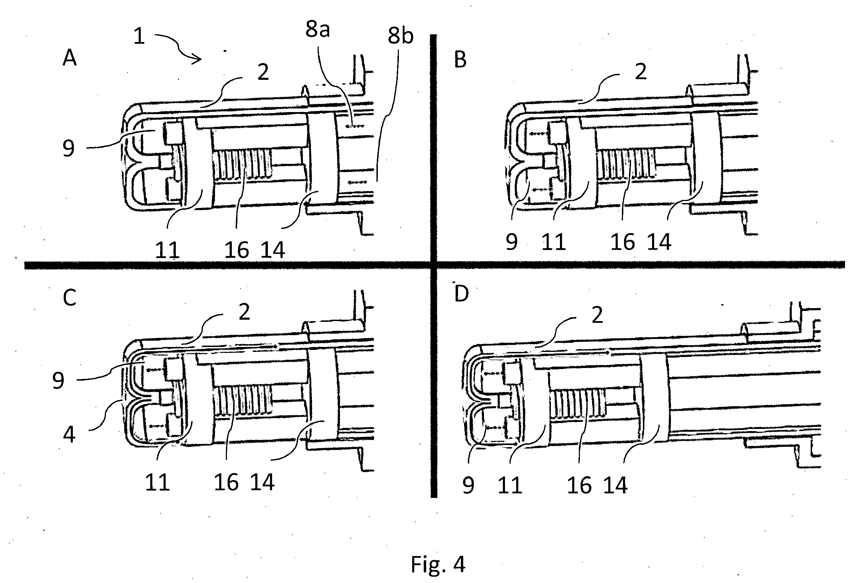

Figure 4 shows the individual steps of the deployment method of the tubular sheath 2 in the insertion instrument 1a according to the first embodiment. In a first step A, air is supplied through the hoses 8a and 8b, reaching the dynamic pressure chamber 9 in step B. When the dynamic pressure chamber 9 is pressurized as in step C, the flexible tubular sheath 2 will be deployed from the reservoir 16 and the distal annular end 4 towards the directions of the proximal annular end (not shown) by sliding on the cylinders 11 and 14 and thus extending the insertion instrument (step D) .

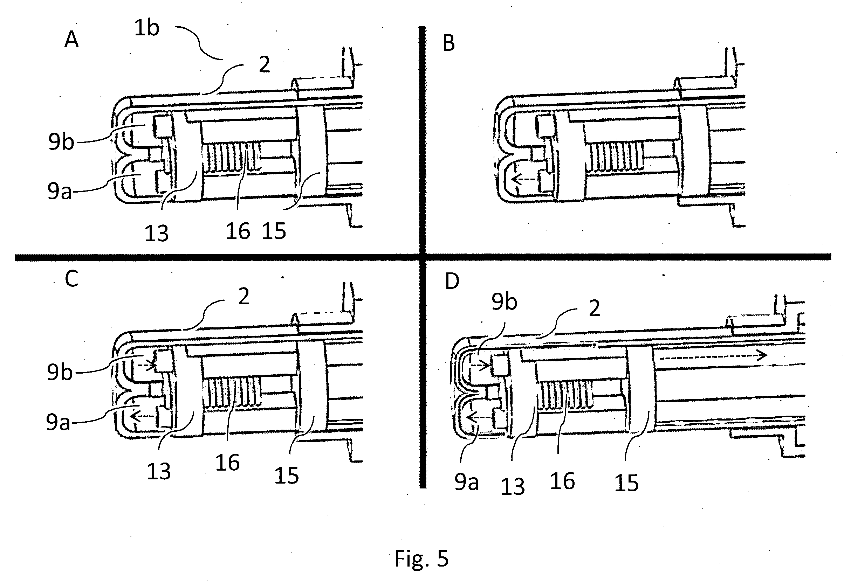

Figure 5 shows individual steps, of the deployment method of the tubular sheath 2 in the insertion instrument 1b according to the second embodiment. The deployment method is similar to the one described with regard to Figure 4 with the difference that one chamber 9a is pressurized and the second chamber 9b is depressurized (step C), thus air is extracted from chamber 9b. The dynamic pressure chambers 9a and 9b can be pressurized independently thus also allowing bending movements of the tip.

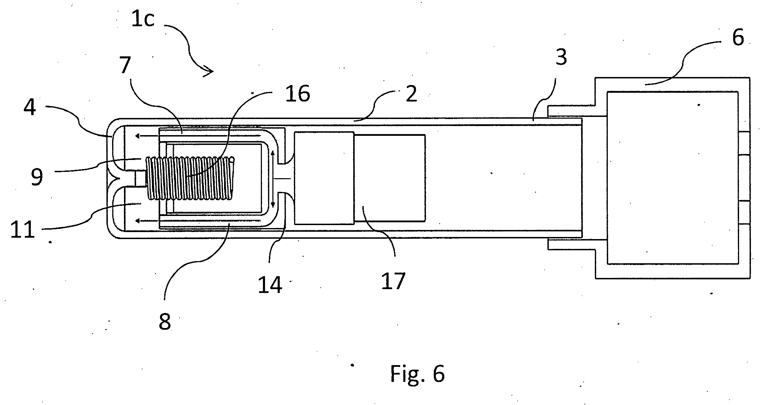

Figure 6 shows a third embodiment of an insertion instrument 1c according to the invention in a sectional view. The insertion instrument 1c is equipped with an integrated fluid supply member 17. An integrated air supply member 17 avoids potential friction with that can occur with hoses. The integrated fluid supply member can have a distal air pump, or a canister/reservoir to store the fluid, e.g. air. It can be directly associated with the pressure transmittal head 7 via the second cylinder 14. The fluid transmission path 8 consist of pipes formed between the first hollow cylinder 11 and the second cylinder 14, transporting air to the dynamic pressure chamber 9 (indicated by the arrows).

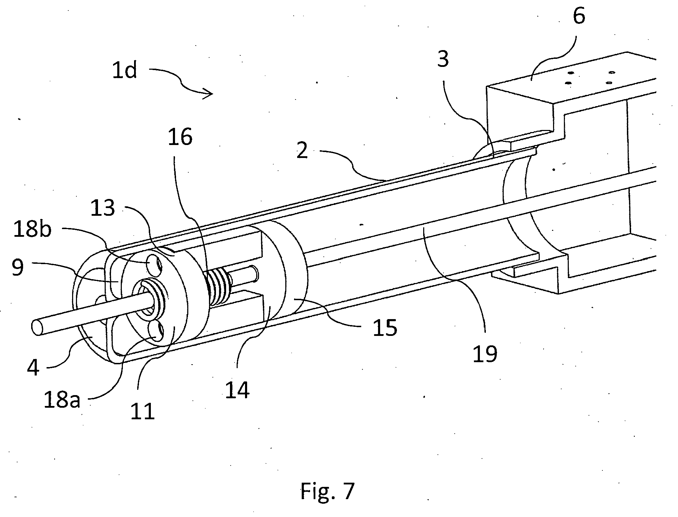

Figure 7 shows a perspective view of a fourth embodiment of an insertion instrument 1d according to the invention. This embodiment is additionally equipped with an interior device channel 19 through which an interior device for examination, e.g. a camera, can be guided to the distal annular end 4 of the insertion instrument 1d.

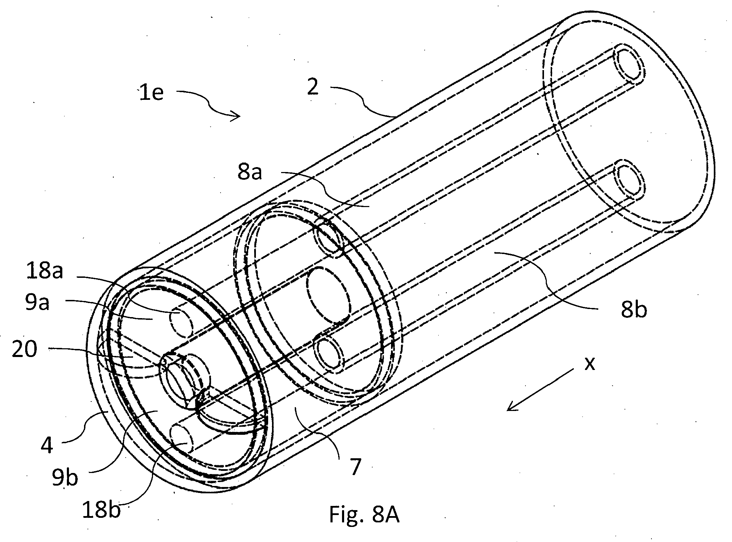

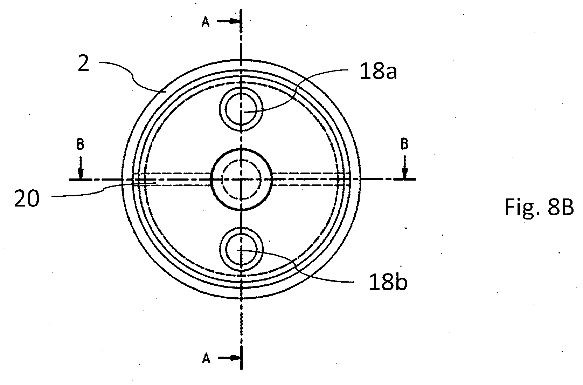

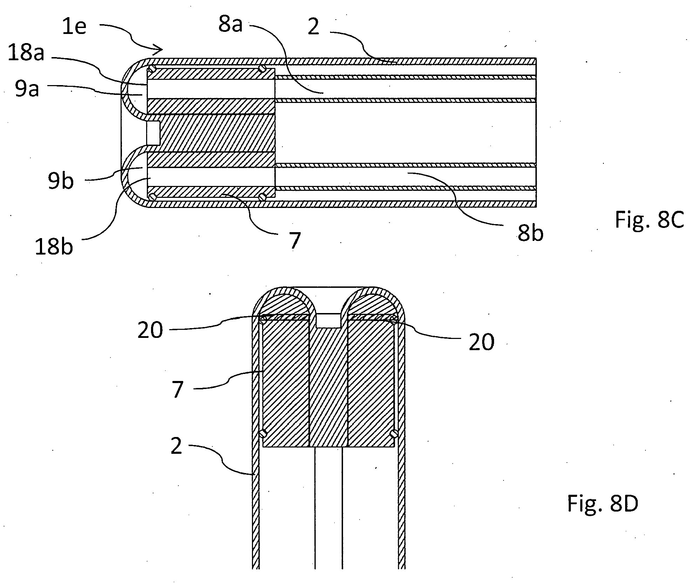

Figures 8A-C show a fifth embodiment of an insertion instrument 1e according to the invention in a perspective view (figure 8A), in a front view (figure 8B), a first sectional view along axis A-A in figure 8B (figure 8C) and a second sectional view along axis B-B in figure 8B (figure 8D).

The main difference compared to the previous described embodiments is a sealing element 20 in the head 7, enabling and/or improving the pressure separation of the chambers 9a and 9b for steering. This could be achieved by sealing element which is highly compliant in the axial direction x, maintaining contact with the semi-toroidal geometry of the everting material 2. For instance, soft silicone ribs or spring-loaded extendable rubber ribs can be used, which extend out of the head 7 along the axial direction x, maintaining constant force on the everting material 2.

It goes without saying that the individual components of the various embodiments can be interchanged and combined with each other, depending on the desired application.

## Claims

1. Insertion instrument (1a-1e), in particular a surgical insertion instrument, for insertion into a cavity, preferably a human cavity, the instrument (1a-1e) comprising
- a flexible tubular sheath (2) having a proximal annular end (3), a distal annular end (4) and a middle section (5) extendable between the proximal annular end (3) and the distal annular end (4),
- a holding member (6) holding the proximal annular end (3),
- a pressurizing unit for pressurizing the distal annular end (4) of the flexible tubular sheath (2) for everting the sheath (2) at the distal annular end (4), wherein the pressurizing unit comprises
- a fluid supply member and
- a pressure transmittal head (7) being movably arranged in the tubular sheath (2) and in proximity to the distal annular end (4), preferably at the distal annular end (4), wherein the pressure transmittal head (7) is in fluid connection with the fluid supply member by means of a fluid transmission path (8),
**characterized in that** the distal annular end (4) and the pressure transmittal head (7) form at least one dynamic pressure chamber (9, 9a, 9b), wherein the flexible tubular sheath (2) is deployable and invertible at the distal annular end (4) upon pressurizing said at least one dynamic pressure chamber (9, 9a, 9b) by means of the pressure transmittal head (7), thereby extending the middle section (5) of the flexible tubular sheath (2).

2. Insertion instrument (1a-1e) according to claim 1, wherein the fluid transmission path (8) comprises at least one, preferably two, unrollable hose(s) (8a, 8b) connected to the pressure transmittal head (7) for delivering fluid from the fluid supply member to the pressure transmittal head (7) .

3. Insertion instrument (1a-1e) according to claim 2, wherein the unrollable hose(s) (8a, 8b) is/are arranged on a rotatable base station (10).

4. Insertion instrument (1a-1e) according to one of the previous claims, wherein the pressure transmittal head (7) comprises a hollow cylinder (11) with a first inner surface (12) and a first outer surface (13) and wherein the flexible tubular sheath (2) is slidable arranged over the first inner surface (12) and the first outer surface (13) to slide towards the proximal end (3).

5. Insertion instrument (1a-1e) according to claim 4, wherein the flexible tubular sheath (2) is sealingly arranged to the first inner surface (12) and to the first outer surface (13) to form the at least one dynamic pressure chamber (9).

6. Insertion instrument (1a-1e) according to one of the previous claims, wherein the pressure transmittal head (7) comprises a second cylinder (14) with a second outer surface (15) and wherein the flexible tubular sheath (2) is slidable arranged over the second outer surface (15) to slide towards the proximal end (3).

7. Insertion instrument (1a-1e) according to claim 6, wherein the flexible tubular sheath (2) is sealingly arranged over the second outer surface (15) to form the at least one dynamic pressure chamber (9, 9a, 9b).

8. Insertion instrument (1a-1e) according to one of the previous claims, wherein the distal annular end (4) and the pressure transmittal head (7) form at least two dynamic pressure chambers (9a, 9b) which are pressurizable with different pressures.

9. Insertion instrument (1a-1e) according to one of the previous claims, wherein the instrument (1a-1e) further comprises a fluid extraction unit.

10. Insertion instrument (1a-1e) according to claim 9 wherein the fluid extraction unit comprises at least one fluid extraction chamber and at least one fluid extraction member in fluid connection with the at least one fluid extraction chamber.

11. Insertion instrument (1a-1e) according to claim 10, wherein the at least one dynamic pressure chamber (9) is in fluid connection with the at least one fluid extraction chamber.

12. Insertion instrument (1a-1e) according to one of the previous claims, wherein the flexible tubular sheath (2) is made of a plastic film, preferably LDPE or LLDPE; a coated fabric or a flexible resin.

13. Insertion instrument (1a-1e) according to one of the previous claims, wherein the non-deployed tubular sheath (2) is wounded up inside a separate reservoir (16), preferably within the pressure transmittal head (7).

14. Insertion instrument (1a-1e) according to one of the previous claims, wherein the flexible tubular sheath (2) has a diameter in the range of 0.1 to 0.8 cm for endovascular use and 0.1 to 2.5 cm for endoscopy.

15. Insertion instrument (1a-1e) according to one of the previous claim wherein a lumen (19) is provided at least in the centre of the distal annular end (4) for accommodating an interior device, preferably a surgical, therapeutic or diagnostic device, sensors, robots, in particular autonomous robots or magnetically guided robots, cameras and drug delivery systems.

16. Set for insertion into a cavity, preferably a human cavity, the set comprising an insertion instrument (1a-1e) as claimed in one of claims 1 to 15 and at least one interior device, preferably provided at a distal annular end (4) of the insertion instrument (1a-1e), wherein the interior device is in particular selected from the group consisting of surgical, therapeutic or diagnostic instruments, sensors, robots, in particular autonomous robots or magnetically guided robots, cameras and drug delivery systems.
